# EUROPÄISCHE PATENTANMELDUNG

(11) **EP 1 107 001 A2**
(43) Veröffentlichungstag der Anmeldung: **13.06.2001**
(21) Anmeldenummer: 00120348.8
(22) Anmeldetag: 16.09.2000
(51) Int. Cl.: G01N 33/38, G01N 11/00, G01N 3/00

(54) **Ausbreittisch**

(30) Priorität: 02.12.1999 DE 19958071; 18.02.2000 DE 10007612
(71) Anmelder: Arnold, Gerd H., 79793 Wutöschingen / Schwerzen (DE)
(72) Erfinder: Arnold, Gerd H., 79793 Wutöschingen / Schwerzen (DE)
(74) Vertreter: Weiss, Peter, Dr.

(57) **Zusammenfassung**

Bei einem Ausbreittisch zum Bestimmen der Konsistenz eines fliessfähigen Materials, insbesondere Frischbeton mit zumindest einer Platte (1.1, 1.2) auf welcher das Material, insbesondere der Frischbeton durch Formänderungsarbeit zu einem Kuchen (8) verfliesst, soll zur Ermittlung und/oder Bestimmung eines Durchmessers (D1, D2) des Kuchens (8) der Platte (1.1, 1.2) wenigstens ein Sensorelement (9.1 bis 9.4) zugeordnet sein.

## Beschreibung

Die Erfindung betrifft einen Ausbreittisch zum Bestimmen der Konsistenz eines fliessfähigen Materials, insbesondere Frischbeton mit zumindest einer Platte auf welcher das Material, insbesondere der Frischbeton durch Formänderungsarbeit zu einem Kuchen verfliesst.

Ein derartiger Ausbreittisch dient zur Bestimmung der Konsistenz eines fliessfähigen Materials, insbesondere Frischbeton und ist bspw. in der Deutschen Industrie Norm DIN 1048 beschrieben, auf welche ausdrücklich verwiesen wird.

Ein herkömmlicher Ausbreittisch steht auf einem festen vorzugsweise waagrechten Untergrund und weist zwei Platten auf.

Die erste Platte wird vor dem Versuch feucht abgewischt anschliessend wird eine kegelartige Form mit stirnseitigen Öffnungen mittig aufgestellt und ggf. über Halteflächen vor verschieben gesichert.

Das Material wird dann lose in die kegelstumpfartige Form eingefüllt, bspw. mittels einer Schaufel und mit Hilfe eines Holzstabes in der Form ohne Verdichtungseinwirkung eingegeben. Anschliessend wird die Form nach oben lotrecht abgezogen und die Platte wird innerhalb von 15 Sekunden, 15 mal, bis sie zu einem Begrenzungselement, insbesondere einem Anschlag hochgehoben und wieder fallengelassen. Anschliessend verfliesst das Material innerhalb von 90 Sekunden zu einem Kuchen, dessen Durchmesser als Ausbreitmass mittels eines Metermasses oder eines Massbandes gemessen wird.

Ist der Kuchen ungleichförmig, so werden mehrere Messungen des Durchmessers vorgenommen und daraus das Mittel gebildet. Anhand des Durchmessers kann auf entsprechende Konsistenzbereiche KS gilt für steif, KP gilt für plastisch, KR gilt für weich und KF gilt für fliessend, rückgeschlossen werden. Hierdurch lässt sich äusserst aufwendig die Konsistenz des Materials, insbesondere des Frischbetons bestimmen.

Nachteilig daran ist, dass durch manuelle Messfehler, Rechenfehler od. dgl. sich Ungenauigkeiten ergeben, die ggf. zu falschen Konsistenzbereichen führen.

Zudem ist ein derartiges Messen sowie Bestimmen eines Konsistenzbereiches, eines Materials zeit- und kostenaufwendig. Zudem können lediglich die o.g. Konsistenzbereiche des Ausbreittisches bestimmt werden.

Daher ist auch eine Anwendungsmöglichkeit auf das manuelle Messen dieser Bereiche beschränkt.

Der vorliegenden Erfindung liegt die Aufgabe zugrunde einen Ausbreittisch der eingangs genannten Art zu schaffen, welcher die genannten Nachteile beseitigt und mit welchem auf einfache, sehr exakte und kostengünstige, genaue schnelle Weise eine Bestimmung von Konsistenz von fliessfähigem Material, insbesondere von Frischbeton möglich ist.

Zur Lösung dieser Aufgabe führt dass zur Ermittlung und/oder Bestimmung eines Durchmessers des Kuchens der Platte wenigstens ein Sensorelement zugeordnet ist.

Bei der vorliegenden Erfindung ist zumindest ein Sensorelement, welches aus einzelnen Sensoren bestehen kann, der Platte vorzugsweise ausgehend von einer Plattenmitte zu einer Aussenkannte zugeordnet. Dabei können eine Mehrzahl von Sensorelemente bspw. kreuzartig, dreieckartig, linien- oder schlangenartig in die Platte eingesetzt sein.

Auch liegt im Rahmen der vorliegenden Erfindung einzelne Sensoren bspw. sternförmig, kreisringartig um die Plattenmitte anzuordnen, um anschliessend einen Durchmesser des verflossenen Materials automatisch nach einer bestimmten, insbesondere bestimmbaren Zeit zu bestimmen.

Anhand der entsprechenden Signale, die durch entsprechendes Überdecken der Sensoren durch das Material, insbesondere durch den Kuchen erzeugt werden, lässt sich in einer Anzeigeeinrichtung, die externer oder interner Bestandteil der Platte, insbesondere des Ausbreittisches sein kann, der Durchmesser vorzugsweise gemittelt, exakt bestimmen.

Entsprechend wird dann in der Anzeigeeinrichtung oder einer Steuereinrichtung der ermittelte und ggf. gemittelte Durchmesser je nach Anzahl der Sensorelemente bzw. Sensoren in einen entsprechenden Konsistenzbereich umgerechnet und an einem Display ggf. optisch mittels Leuchten anzeigt. Dabei sind die einzelnen Konsistenzbereiche frei wählbar, insbesondere bestimmbar und beliebig unterteilbar.

Ferner hat sich als besonderes vorteilhaft erwiesen der Platte ein Feuchtemesssondenelement sowie ein Temperatursensor zuzuordnen, die entsprechende absolute Feuchtigkeitswerte und Temperaturwerte an die Anzeigeeinrichtung liefern. Hierdurch können zusätzliche Informationen über die Konsistenz, insbesondere über die Materialeigenschaften des gemessenen Materials, insbesondere Frischbetons ermittelt werden.

Die Anzeigeeinrichtung kann solar- und/oder akkubetrieben als interner Bestandteil der Platte zugeordnet sein. Auch kann sie als externer Bestandteil ebenfalls verbunden mit einer Steuerung an die Platte, insbesondere an den Ausbreittisch anschliessbar sein.

Auch sei daran gedacht, an die Platte und/oder an die Anzeigeeinrichtung eine Schnittstelle, die ggf. mit einem Personal-Computer verbunden werden kann, anzuschliessen, um entsprechende Daten abzuspeichern und später ggf. bei erneuten Messungen als Vergleichswerte zu verwenden.

Zudem hat sich als günstig erwiesen die Platte mit entsprechenden Libellen, wasserwaagenartig zu versehen, damit ein Ausrichten, was herkömmlich mittels Wasserwaagen erfolgt, zu erleichtern.

Insgesamt ist mit der vorliegenden Erfindung ein Ausbreittisch geschaffen, welcher universell einsetzbar ist und mit welchem auf sehr schnelle und exakte Weise im Konsistenzbereich eine absolute Feuchtigkeit und eine Temperatur eines Materials, insbesondere Frischbetons kostengünstig und schnell bestimmt werden kann.

Weitere Vorteile, Merkmale und Einzelheiten der Erfindung ergeben sich aus der nachfolgenden Beschreibung bevorzugter Ausführungsbeispiele sowie anhand der Zeichnung; diese zeigt in
Figur 1 eine perspektivisch dargestellte Ansicht eines herkömmlichen Ausbreittisches mit kegelstumpfförmiger Form und Holzstab zum Verteilen von Material, insbesondere Beton;
Figur 2 eine Draufsicht auf einen erfindungsgemässen Ausbreittisch zum Bestimmen der Konsistenz eines flüssigen Materials;
Figuren 3a bis 3d weitere Ausführungsbeispiele möglicher Anordnungen von entsprechenden Sensorelementen auf der Platte des Ausbreittisches gemäss Figur 2;
Figur 4 eine Draufsicht auf ein weiteres Ausführungsbeispiel eines Ausbreittisches mit externer Anzeigeeinrichtung;
Figur 5 eine Draufsicht auf einen Ausbreittisch gemäss den Figuren 2 bis 4 in einer möglichen Gebrauchslage;
Figur 6 ein weiteres Ausführungsbeispiel des Ausbreittisches gemäss den Figuren 2 bis 3d mit Libelle.

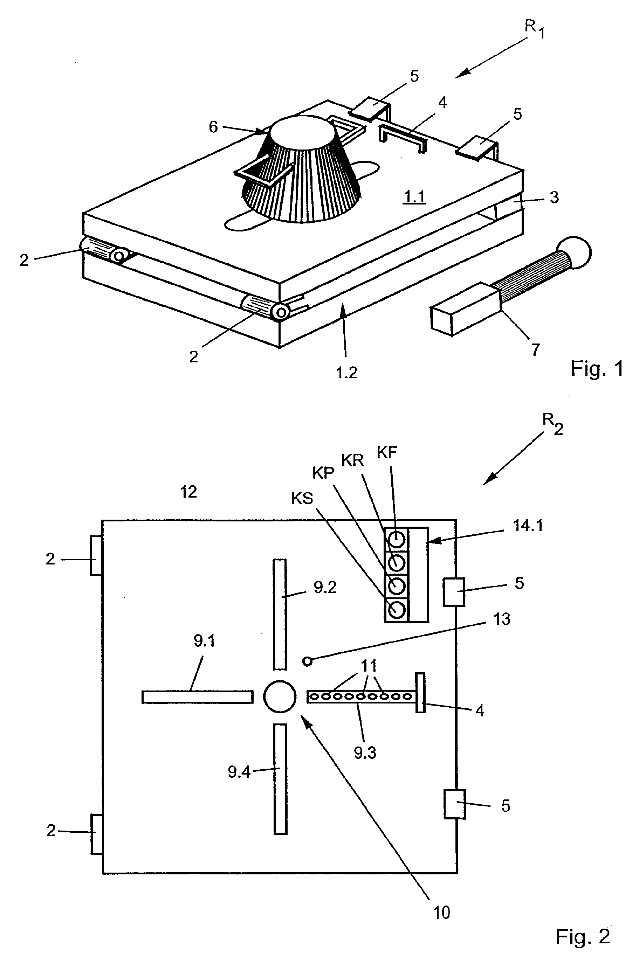

Gemäss Figur 1 weist ein Ausbreittisch R₁, wie er im Stand der Technik bekannt ist, eine erste Platte 1.1 auf, die gegenüber einer darunterliegenden Platte 1.2 über zumindest ein Gelenk 2 miteinander vorzugsweise parallel verbunden ist.

Dabei sind die Platten 1.1., 1.2 über zumindest ein Distanzstück 3 zueinander beabstandet.

Der Platte 1.1 ist ein Halteelemente 4 zugeordnet und dient zum Anheben der Platte 1.1 gegenüber der Platte 1.2.

Eine Bewegung der Platte 1.1, um das Gelenk 2 wird durch Begrenzungselemente 5 und beschränkt zugelassen. Mittels des Halteelementes 4 lässt sich die Platte 1.1 gegenüber der unten liegenden Platte 1.1 in herkömmlicher Weise rütteln.

Auf die Platte 1.1 wird eine kegelstumpfartige Form 6, die oben und unten geöffnet ist, aufgestellt. In diese Form 6 wird fliessfähiges Material, insbesondere Frischbeton eingefüllt und mittels eines Holzstabes 7 verteilt.

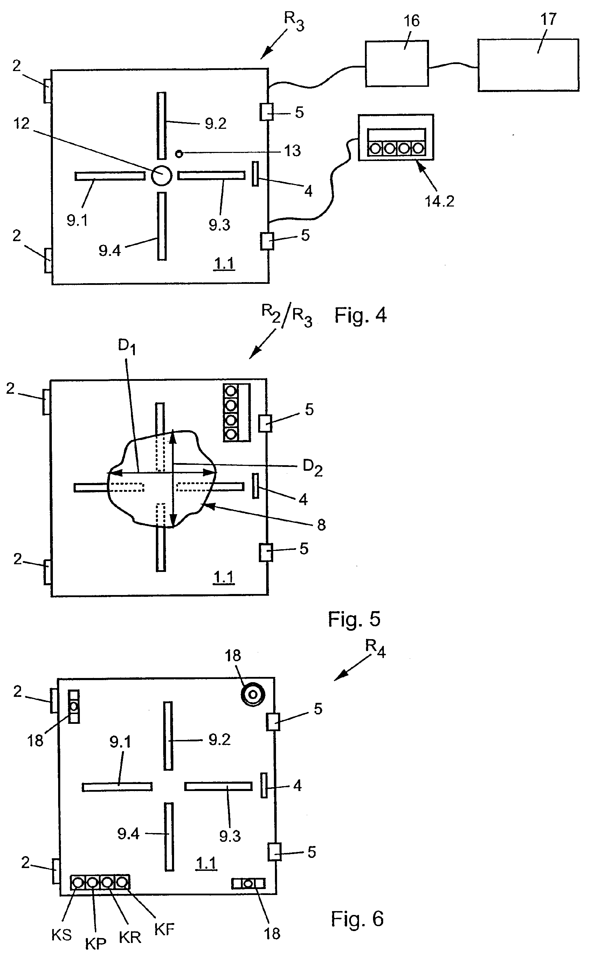

Anschliessend wird die Form 6 lotrecht nach oben abgezogen und das Material, insbesondere der Frischbeton, verfliesst innerhalb einer bestimmten Zeit zu einem Kuchen 8, wie er insbesondere in Figur 5 angedeutet ist. Durch die entsprechende Formänderung vom kegelstumpfartigen Beton aus der Form 6 zu einem Kuchen 8 innerhalb einer bestimmten Zeit lassen sich bspw. die Verformbarkeit, die Beweglichkeit und die Verdichtungswilligkeit des Materials ermitteln.

Insbesondere in der Deutschen Industrie Norm DIN 1048 ist ein Prüfverfahren für Beton mit einem entsprechenden Ausbreittisch beschrieben. Hierauf wird ausdrücklich Bezug genommen.

Anhand der Grösse des Kuchens 8, insbesondere des Durchmessers D1, D2, lässt sich ein Konsistenzbereich, bspw. KS für steif, KP für plastisch KR für weich, KF für fliessend ermitteln. Dabei wird in herkömmlicher Weise der Durchmesser des Kuchens 8 mittels eines Meterstabes, Massbandes od. dgl. abgemessen und über entsprechende Tabellen der entsprechende Konsistenzbereich abgelesen bzw. ermittelt.

Nachteilig ist, dass das Messen des Durchmessers von Hand sehr aufwendig und ungenau ist, so dass die Konsistenzbereiche nicht genau, exakt und schnell zu bestimmen sind.

In dem Ausführungsbeispiel der vorliegenden Erfindung gemäss Figur 2 ist einer Platte 1.1 eines Ausbreittisches R₂ zumindest ein Sensorelement 9.1 bis 9.4 vorzugsweise kreuz- oder sternartig um eine Plattenmitte 10 angeordnet. Bevorzugt sind die einzelnen Sensorelemente 9.1 bis 9.4 aus einer Mehrzahl von einzelnen Sensoren 11 gebildet, die ein unterschiedliches Ausbreiten des Materials, insbesondere des Kuchens 8 erkennen. Je nach Ausbreitung des Materials bekommen die einzelnen Sensoren 11 der Sensorelemente 9.1 bis 9.4 entsprechende Signale, aufgrund dessen ein exakter Durchmesser D1, D2 des Kuchens 8, ermittelt werden kann.

Vorzugsweise mittig in der Plattenmitte 10 ist ein Feuchtemesssondenelement 12 sowie ein Temperatursensor 13 angeordnet, wobei über das Feuchtemesssondenelement 12 direkt die absolute Feuchtigkeit des Materials, insbesondere des Betons bestimmbar ist und über den Temperatursensor 13 eine Temperatur exakt ermittelbar ist.

Es hat sich ferner als günstig erwiesen, der Platte 1.1 eine Anzeigeeinrichtung 14.1 zuzuordnen, in welcher der aus dem ggf. gemittelten Durchmesser ermittelte Konsistenzbereich KS, KP, KR und KF oder eine entsprechende andere wählbare Unterteilung bspw. optisch durch Anzeigeleuchten ablesbar ist.

Ebenso kann in einem Display die Temperatur wie auch die absolute Feuchtigkeit, ggf. verglichen mittels einer abgelegten und abgespeicherten Eichkurve angezeigt werden.

Bevorzugt wird die Anzeigeeinrichtung 14.1 akku- und/oder solarbetrieben.

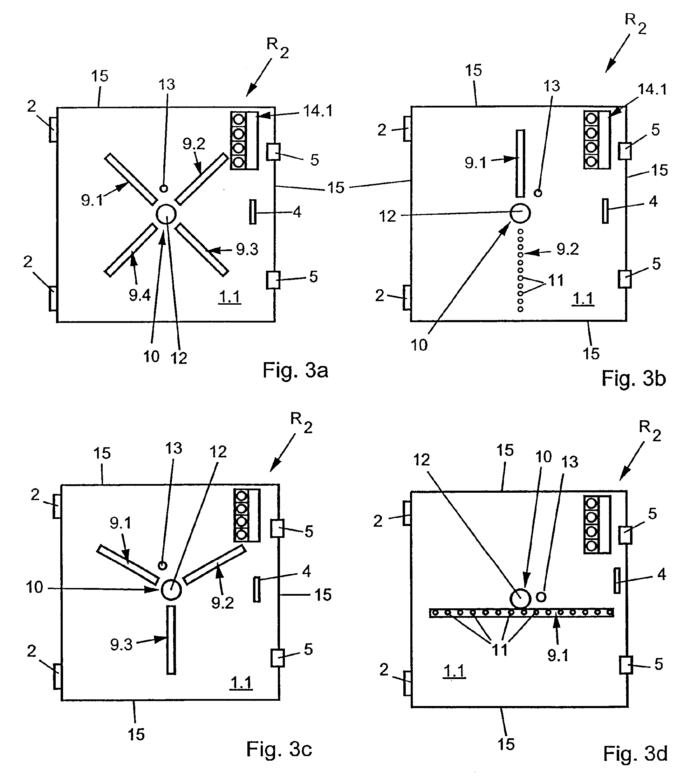

Wie insbesondere in den Figuren 3a bis 3d dargestellt ist, können die einzelnen Sensorelemente 9.1 bis 9.4 einzelnen, insbesondere im Bereich der Plattenmitte 10 angeordnet sein, wobei diese sich sternartig, linienartig oder schlangenartig vorzugsweise zu einer Aussenkante 15 der Platte 1.1 hin erstrecken.

Die Anordnung der einzelnen Sensorelemente 9.1 bis 9.4 ist nur beispielhaft beschrieben. Hier sind unterschiedliche Anordnungen der Sensorelemente 9.1 bis 9.4 sowie der Einzelsensoren 11 denkbar.

Beispielsweise soll auch im Rahmen der Erfindung liegen, kreisförmig angeordnete einzelne Sensoren 11 oder streifenartige Sensoren 11 der Plattenmitte 10, vorzugsweise nach aussen erstreckend anzuordnen.

In dem Ausführungsbeispiel der vorliegenden Erfindung gemäss Figur 4 ist der Ausbreittisch R₃ in oben beschriebener Weise dargestellt, wobei diesem eine externe Anzeigeeinrichtung 14.2 zum Anzeigen der Konsistenzbereiche KS, KP, KR, KF oder anderen Werten, wie Feuchtigkeitswerte und Temperaturen des Materials zugeordnet ist.

Diese steht indirekt oder direkt mit den Sensorelementen 9.1 bis 9.4 sowie dem Feuchtemesssondenelement 12 und dem Temperatursensor 13 in Verbindung. Ebenso kann eine Schnittstelle 16 direkt mit der Anzeigeeinrichtung 14.2 oder in oben beschriebender Weise mit dem Ausbreittisch R₃ verbunden sein, die wiederum mit einem Personal-Computer 17 zur Auswertung verbunden sein kann.

Hierzu lassen sich die ermittelten Werte, Messwerte bspw. einer Messserie abspeichern, bearbeiten und ggf. als Vergleichswerte im Anzeigefeld wieder darstellen. Bei erneuten Messungen können sie als Vergleichswerte dienen.

In den Figuren 5 und 6 ist ein Ausbreittisch R4 aufgezeigt, in dessen Platte 1.1 eine Mehrzahl von Wasserwagen und/oder Libellen 18 ggf. unterschiedlicher Bauweise eingesetzt sein können, um insgesamt den Ausbreittisch R4 gegenüber jedem beliebigen Untergrund waagrecht auf einfache genaue Weise auszurichten.

Ferner liegt im Rahmen der vorliegenden Erfindung, dass beispielsweise über die Anzeigeeinrichtung 14 eine Zeit messbar ist, wobei die Zeitmessung mit dem Aufbringen des fliessfähigen Materials, insbesondere Betons auf die Platte 1.1, 1.2 beginnt.

Ist ein bestimmbarer und gewählter, ggf. genormter Durchmesser (D1, D2), insbesondere auch gemittelter Durchmesser, welcher ggf. über die Sensorelemente 9.1 bis 9.4 bestimmt und errechnet werden kann, erreicht, so wird die Zeitmessung automatisch beendet. Die Dauer, insbesondere die ermittelte Zeit seit dem Aufbringen des fliessfähigen Materials auf die Plappe 1.1, 1.2 bis zum Erreichen eines vorherbestimmten und gewählten Durchmessers D1, D2 wird ermittelt.

Anhand dieser Dauer bzw. dieser Zeit lässt sich unmittelbar auf die Konsistenzbereiche (KS, KP, KR, KF) schliessen. Die Anzeige und Umrechnung erfolgt analog in oben beschriebener Weise. Auch liegt im Rahmen der vorliegenden Erfindung, eine bestimmte Zeit oder Dauer vorherzubestimmen bzw. vorzugeben, in welcher das fliessfähige Material, insbesondere der Beton verfliesst.

Ist die Zeit seit dem Aufbringen des fliessfähigen Materials auf die Platte 1.1, 1.2 und dem anschliessenden Verfliessen erreicht bzw. abgelaufen, so wird mit Zeitablauf zu diesem Zeitpunkt der tatsächlich verflossene Durchmesser D1, D2 bestimmt, wobei anhand des tatsächlich verflossenen Durchmessers, insbesondere ggf. gemittelten abgelesenen oder in oben beschriebener Weise ermittelten Durchmessers ein unmittelbarer Rückschluss auf den Konsistenzbereich KS, KP, KR, KF ermöglicht, insbesondere umgerechnet wird und in oben beschriebener Weise anzeigbar ist.

Ferner ist auch denkbar, dass permanent eine Zeitmessung seit Beginn des Aufbringens des fliessfähigen Materials auf die Platte erfolgt und gleichzeitig permanent eine Messung mittels der o.g. Sensorelemente 9.1 bis 9.2 des Durchmessers, insbesondere gemittelt erfolgt, so dass permanent über den permanenten Fliessprozess eine Aussage über den Konsistenzbereich getroffen werden kann.

Dabei kann, wie oben beschrieben, eine abschliessende Messung und Auswertung, beispielsweise nach Erreichen eines vorgegebenen Durchmessers oder bei Erreichen einer bestimmten vorgegebenen Zeit erfolgen.

Auch ist denkbar über einen kontinuierlichen Verfliessprozess die Konsistenz des fliessfähigen Materials, insbesondere die Viskosität permanent anzuzeigen und zu bestimmen.

| **Positionszahlenliste** | | | | | |
|---|---|---|---|---|---|
| 1 | Platte | 34 | | 67 | |
| 2 | Gelenk | 35 | | 68 | |
| 3 | Distanzstück | 36 | | 69 | |
| 4 | Halteelement | 37 | | 70 | |
| 5 | Begrenzungs- element | 38 | | 71 | |
| 6 | Form | 39 | | 72 | |
| 7 | Holzstab | 40 | | 73 | |
| 8 | Kuchen | 41 | | 74 | |
| 9 | Sensorelement | 42 | | 75 | |
| 10 | Plattenmitte | 43 | | 76 | |
| 11 | Sensor | 44 | | 77 | |
| 12 | Feuchtemess- sondenelement | 45 | | 78 | |
| 13 | Temperatursensor | 46 | | 79 | |
| 14 | Anzeigeein- richtung | 47 | | | |
| 15 | Aussenkante | 48 | | | |
| 16 | Schnittstelle | 49 | | R₁ | Ausbreittisch |
| 17 | PC | 50 | | R₂ | Ausbreittisch |
| 18 | Libelle | 51 | | R₃ | Ausbreittisch |
| 19 | | 52 | | R₄ | Ausbreittisch |
| 20 | | 53 | | | |
| 21 | | 54 | | D1 | Durchmesser |
| 22 | | 55 | | D2 | Durchmesser |
| 23 | | 56 | | | |
| 24 | | 57 | | | |
| 25 | | 58 | | KS | Konsistenzbereich |
| 26 | | 59 | | KP | Konsistenzbereich |
| 27 | | 60 | | KR | Konsistenzbereich |
| 28 | | 61 | | KF | Konsistenzbereich |
| 29 | | 62 | | | |
| 30 | | 63 | | | |
| 31 | | 64 | | | |
| 32 | | 65 | | | |
| 33 | | 66 | | | |

## Patentansprüche

1. Ausbreittisch zum Bestimmen der Konsistenz eines fliessfähigen Materials, insbesondere Frischbeton mit zumindest einer Platte (1.1, 1.2) auf welcher das Material, insbesondere der Frischbeton durch Formänderungsarbeit zu einem Kuchen (8) verfliesst,
dadurch gekennzeichnet,
dass zur Ermittlung und/oder Bestimmung eines Durchmessers (D1, D2) des Kuchens (8) der Platte (1.1) wenigstens ein Sensorelement (9.1 bis 9.4) zugeordnet ist.

2. Ausbreittisch zum Bestimmen der Konsistenz eines fliessfähigen Materials, insbesondere Frischbeton mit zumindest einer Platte (1.1, 1.2) auf welcher das Material, insbesondere der Frischbeton durch Formänderungsarbeit zu einem Kuchen (8) verfliesst, dadurch gekennzeichnet, dass zur Ermittlung und/oder Bestimmung der Konsistenz eines fliessfähigen Materials die Zeit bis zum Erreichen eines wählbaren Durchmessers (D1, D2) ermittelbar ist oder dass zur Ermittlung und/oder Bestimmung der Konsistenz eines fliessfähigen Materials innerhalb einer vorgegebenen Zeit ein Durchmesser (D1, D2) des Kuchens (8) bestimmbar ist.

3. Ausbreittisch zum Bestimmen der Konsistenz eines fliessfähigen Materials, insbesondere Frischbeton mit zumindest einer Platte (1.1, 1.2) auf welcher das Material, insbesondere der Frischbeton durch Formänderungsarbeit zu einem Kuchen (8) verfliesst, dadurch gekennzeichnet, dass zur Ermittlung und/oder Bestimmung einer Temperatur des Kuchens (8) der Platte (1.1, 1.2) wenigstens ein Temperatursensor (13) zugeordnet ist.

4. Ausbreittisch zum Bestimmen der Konsistenz eines fliessfähigen Materials, insbesondere Frischbeton mit zumindest einer Platte (1.1, 1.2) auf welcher das Material, insbesondere der Frischbeton durch Formänderungsarbeit zu einem Kuchen (8) verfliesst, dadurch gekennzeichnet, dass zur Ermittlung und/oder Bestimmung einer absoluten Feuchtigkeit des Materials, der Platte (1.1, 1.2), zumindest ein Feuchtemesssondenelement (12) zugeordnet ist.

5. Ausbreittisch nach wenigstens einem der Ansprüche 1 bis 4, dadurch gekennzeichnet, dass der Platte (1.1, 1.2) zumindest eine Anzeigeeinrichtung (14.1, 14.2) zugeordnet oder an diese als externer Bestandteil anschliessbar ist.

6. Ausbreittisch nach Anspruch 5, dadurch gekennzeichnet, dass an der Anzeigeeinrichtung (14.1, 14.2) ein ermittelter ggf. gemittelter Durchmesser (D1, D2) des Kuchens (8) und/oder daraus resultierender Konsistenzbereich (KS, KP, KR, KF) ermittelbar und/oder anzeigbar ist.

7. Ausbreittisch nach Anspruch 5 oder 6, dadurch gekennzeichnet, dass an der Anzeigeeinrichtung (14.1, 14.2) eine Temperatur oder ein Feuchtigkeitswert des Materials anzeigbar und/oder ermittelbar ist, wobei ggf. die Feuchtigkeitswerte mit abgespeicherten Eichkurven und/oder Toleranzfenster vergleichbar sind.

8. Ausbreittisch nach wenigstens einem der Ansprüche 1 bis 7, dadurch gekennzeichnet, dass der Platte (1.1., 1.2) zumindest eine Libelle (18) zugeordnet ist.

9. Ausbreittisch nach wenigstens einem der Ansprüche 1 bis 8, dadurch gekennzeichnet, dass das zumindest eine Sensorelement (9.1 bis 9.4) zwischen einer Plattenmitte (10) und einer Aussenkante (15) der Platte (1.1, 1.2) angeordnet ist.

10. Ausbreittisch wenigstens einem der Ansprüche 1 bis 9, dadurch gekennzeichnet, dass das zumindest eine Sensorelement (9.1 bis 9.4) aus einer Mehrzahl einzelner Sensoren (11) gebildet ist.

11. Ausbreittisch nach wenigstens einem der Ansprüche 1 bis 10, dadurch gekennzeichnet, dass das zumindest eine Sensorelement (9.1 bis 9.4) aus wenigstens einem Widerstandssensor, Solarsensor, IR-, UV-, Ultraschallsensor, Fotosensor, IR-Vorhang od. dgl. gebildet ist.

12. Ausbreittisch nach wenigstens einem der Ansprüche 1 bis 11, dadurch gekennzeichnet, dass das Sensorelement (9.1 bis 9.4) leistenartig der Platte (1.1, 1.2) zugeordnet ist und um eine Plattenmitte (10) eben ausgerichtet, insbesondere angeordnet ist.

13. Ausbreittisch nach wenigstens einem der Ansprüche 1 bis 12, dadurch gekennzeichnet, dass an die Anzeigeeinrichtung (14.1, 14.2) und/oder an die Sensorelemente (9.1 bis 9.4) eine Schnittstelle (16) zur Datenübertragung auf einen Personal-Computer (17) anschliessbar ist.

14. Ausbreittisch nach wenigstens einem der Ansprüche 1 bis 13, dadurch gekennzeichnet, dass durch Überdeckung der einzelnen Sensorelemente (9.1 bis 9.4) durch das Material, insbesondere den Kuchen (8) überdeckte Sensorelemente (9.1 bis 9.4) in der Anzeigeeinrichtung (14.1, 14.2) ein Signal erzeugen, welches zur Ermittlung des ggf. gemittelten Durchmessers (D1, D2) des Kuchens (8) dient, wobei der ermittelte Durchmesser (D1, D2) des Kuchens (8) auf einen anzeigbaren Konsistenzbereich (KS, KP, KR, KF) schliesst.

15. Ausbreittisch nach wenigstens einem der Ansprüche 2 bis 14, dadurch gekennzeichnet, dass durch eine vorgegebene und bestimmbare Zeit, insbesondere Dauer, unmittelbar nach dem Aufbringen des Frischbetons auf die Platte (1.1, 1.2) ein nach einem Verfliessen resultierender Durchmesser (D1, D2) des Kuchens (8) ggf. mittels den Sensorelementen (9.1, bis 9.4) bestimmbar ist, um hieraus die Konsistenz bzw. den Konsistenzbereich (KS, KP, KR, KF) des fliessfähigen Materials zu bestimmen.

16. Ausbreittisch nach wenigstens einem der Ansprüche 2 bis 14, dadurch gekennzeichnet, dass unmittelbar mit dem Aufbringen des Frischbetons auf die Platte (1.1, 1.2) ggf. automatisch eine Zeitmessung beginnt und nach Erreichen eines vorgegebenen und vorherbestimmten Durchmessers (D1, D2) des Kuchens (8) die Zeit ermittelbar bzw. bestimmbar ist, über welche die Konsistenz des fliessfähigen Materials ermittelbar ist.
